# EUROPÄISCHE PATENTSCHRIFT

(11) **EP 3 210 989 B1**
(45) Veröffentlichungstag und Bekanntmachung des Hinweises auf die Patenterteilung: **26.09.2018**
(21) Anmeldenummer: 16157675.6
(22) Anmeldetag: 26.02.2016
(51) Int. Cl.: C07F 15/00, C07C 211/63, B01J 23/42, C01G 55/00

(54) **EDELMETALLVERBINDUNGEN FÜR TRÄGERGESTÜTZTE KATALYSATOREN**
NOBLE METAL COMPOUNDS FOR SUPPORTED CATALYSTS
COMPOSÉS EN METAUX PRECIEUX POUR CATALYSATEURS SUPPORTÉS

(43) Veröffentlichungstag der Anmeldung: 30.08.2017
(73) Patentinhaber: Umicore AG & Co. KG, 63457 Hanau-Wolfgang (DE)
(72) Erfinder: WOERNER, Eileen, 61130 Nidderau (DE); EBERT, Timo, 63796 Kahl (DE); LENNARTZ, Michael, 60435 Frankfurt (DE); KARCH, Ralf, 63801 Kleinostheim (DE); DOPPIU, Angelino, 63500 Seligenstadt (DE); RIVAS-NASS, Andreas, 64625 Bensheim (DE); FREY, Annika, 63457 Hanau (DE)

(56) Entgegenhaltungen:
- EP-A2- 1 175 948
- CN-A- 103 864 855
- CN-A- 103 880 889
- ZHENGZHENG YANG ET AL: "Size-dependent CO and propylene oxidation activities of platinum nanoparticles on the monolithic Pt/TiO 2 -YO x diesel oxidation catalyst under simulative diesel exhaust conditions", CATALYSIS SCIENCE & TECHNOLOGY, Bd. 5, Nr. 4, 2015, Seiten 2358-2365, XP055295839, United Kingdom ISSN: 2044-4753, DOI: 10.1039/C4CY01384K

## Beschreibung

Bei der Abscheidung von Platinmetall auf Trägermaterialien, wie z.B. Oxidpartikeln, werden an die platinhaltigen Platin-Ausgangsverbindungen mehrere Anforderungen gestellt.

Diese sollen vorteilhaft in basischen Lösungen mit einem Platingehalt von mindestens 10% hergestellt werden können, halogenfrei oder zumindest halogenarm sein, mit Ausnahme von Kohlenstoff, Wasserstoff, Sauerstoff, und Stickstoff keine Fremdelemente enthalten, einen möglichst niedrigen Stickstoffgehalt aufweisen, um Stickoxidemissionen zu minimieren. Zudem sollen sie durch Veränderung des pH Werts möglichst vollständig ausgefällt werden können.

EP 1175948 zeigt ([EtNH₃]₂[PtOH₆]) und dessen Verwendung zur Herstellung trägergestützter Katalysatoren, insbesondere Elektroden-Katalysatoren in Brennstoffzellen.

CN 103 864 855 zeigt [HO-CH₂-CH₂-NH₃] [PtOH₆], abgekürzt als Pt-EAH, sowie ein Herstellungsverfahren aus H₂PtOH₆ und EA in Wasser und die Verwendung von Pt-EAH als Precursor zur Herstellung trägergestützter Katalysatoren.

CN 103 880 889 zeigt ebenfalls die Verbindung Pt-EAH sowie ein Herstellungsverfahren aus H₂PtOH₆ und EA und die Verwendung von Pt-EAH als Precursor zur Herstellung trägergestützter Katalysatoren mit geringem Chlorid- und Nitratgehalt.

Zhenzheng Yang et al., "Size-dependent CO and propylene oxidation activities of platinum nanoparticles on the monolithic Pt/TiO2-YOx diesel oxidation catalyst under simulative diesel exhaust conditions", Catalysis Science & Technology, 2015, Band 5 Nr. 4, 2358-2365 zeigt die Verwendung von Pt-EAH als Precursor zur Herstellung trägergestützter Platin-Oxidationskatalysatoren zur Dieselmotor-Abgasbehandlung.

Diese Vorgaben werden von Tetraethylammonium-hexahydroxoplatinat, (N(CH₂CH₃)₄)₂[Pt(OH)₆], nachfolgend als Pt-TEAH bezeichnet erfüllt.

Die Verbindung kann als Lösung mit der gewünschten Platinkonzentration hergestellt werden. Der Gehalt an Halogen (insbesondere Chlor) wird über den Chlorgehalt der verwendeten Edukte eingestellt.

Pt-TEAH kann durch Umsetzung von Hexahydroxoplatinsäure mit Tetraethylammoniumhydroxid, optional in Gegenwart eines Reaktionsmediums, hergestellt werden.

Hexahydroxoplatinsäure kann mit verschiedenen Halogengehalten, insbesondere Chlorgehalten, hergestellt werden. Diese liegen im Allgemeinen bei 30.000 ppm oder weniger, oder 20.000 ppm oder weniger, oder 15.000 ppm oder weniger, insbesondere 10.000 ppm oder weniger, 5.000 ppm oder weniger oder 1.000 ppm oder weniger, wobei sich der Halogengehalt auf Platinmetall bezieht.

Die Zugabereihenfolge der Edukte Hexahydroxoplatinsäure und Tetraethylammoniumhydroxid ist unkritisch. Daher kann Tetraethylammoniumhydroxid oder deren Lösung vorgelegt und danach Hexahydroxoplatinsäure zugegeben oder aber zunächst Hexahydroxoplatinsäure vorgelegt und anschließend Tetraethylammoniumhydroxid oder deren Lösung zugegeben und anschließend miteinander umgesetzt werden.

Optional kann die Umsetzung in einem Reaktionsmedium erfolgen. Dies kann vorteilhaft ein Lösemittel sein. Gut geeignet sind polare, protische Lösemittel.

Das Reaktionsmedium kann ausgewählt sein aus der Gruppe bestehend aus Wasser, ein oder mehrere Alkohole, eine oder mehrere Säuren und deren Mischungen. Insbesondere kann das Reaktionsmedium ein Lösemittel ausgewählt aus der Gruppe bestehend aus Wasser, Methanol, Ethanol, Propanol, Isopropanol, Butanol, 1-Methylbutanol, 2-Methylbutanol, tert.-Butanol, Ameisensäure, Essigsäure, Propionsäure und deren Kombinationen sein. Besonders geeignet als Reaktionsmedium ist Wasser; in der Praxis wird in diesem Fall üblicherweise demineralisiertes Wasser bzw. destilliertes Wasser verwendet.

Vorteilhaft wird das Reaktionsmedium vor Zugabe der Edukte vorgelegt. Es kann jedoch auch gleichzeitig mit einem Edukt vorgelegt werden, beispielsweise wenn ein Edukt als Lösung oder Suspension vorgelegt oder zugegeben wird, wie zum Beispiel eine Lösung von Tetraethylammoniumhydroxid.

Die Edukte werden für eine vorbestimmte Reaktionszeit und bei einer in einem vorbestimmten Bereich liegenden Reaktionstemperatur umgesetzt.

Im Allgemeinen ist eine Reaktionszeit von 10 Minuten bis 24 Stunden ausreichend, insbesondere eine Reaktionszeit von 20 Minuten bis 12 Stunden, 30 Minuten bis 6 Stunden, 45 Minuten bis 3 Stunden oder 45 Minuten bis 120 Minuten, oder 50 Minuten bis 70 Minuten. Meist ist eine Reaktionszeit von etwa einer Stunde ausreichend.

Die Reaktionstemperatur kann von 10°C bis 100°C, insbesondere 15°C bis 80°C oder 20°C bis 50°C oder 20°C bis 40°C betragen. Wird ein Reaktionsmedium eingesetzt, so liegt die Reaktionstemperatur maximal bei der Siedetemperatur des Reaktionsmediums. In der Praxis bewährt haben sich Temperaturen von 20°C bis 50°C.

Zur Herstellung von Pt-TEAH werden mindestens 1,7 Moläquivalente Tetraethylammoniumhydroxid pro Mol Hexahydroxoplatinsäure eingesetzt. Bewährt haben sich 1,7 Moläquivalente bis 17 Moläquivalente, insbesondere 2 Moläquivalente bis 6 Moläquivalente Tetraethylammoniumhydroxid pro Mol Hexahydroxoplatinsäure.

Das gewünschte Produkt, Pt-TEAH, fällt bei Verwendung eines Reaktionsmediums als Lösung an, insbesondere als wässrige Lösung, wobei die Platinkonzentration von 1 Gew.-% bis 15 Gew.-%, insbesondere 10 Gew.-% bis 15 Gew.-% beträgt.

Pt-TEAH bzw. dessen Zubereitungen können als Precursor zur Herstellung heterogener Katalysatoren (wie z.B. Autoabgaskatalysatoren), für den Einsatz in galvanischen Bädern, als Vorprodukt für die Herstellung homogener Katalysatoren oder auch selbst als homogener Katalysator verwendet werden.

Zur Herstellung eines heterogenen Katalysators kann eine Lösung von Pt-TEAH mit einem Trägermaterial wie einem Metalloxid, wie beispielsweise Aluminiumoxid oder Siliziumdioxid, vermischt werden. Das Metalloxid, also Aluminiumoxid oder Siliziumdioxid, wird vorteilhaft in Form von Pulver oder Partikel eingesetzt.

Anschließend wird das Pt-TEAH umgesetzt, d.h. einer chemischen Reaktion unterworfen, wobei eine Platinverbindung ausfällt und sich zumindest teilweise auf der Oberfläche der Trägermaterialpartikel, also der Metalloxid partikel, abscheidet.

Die Umsetzung und das Ausfällen der Platinverbindung kann durch Änderung des pH-Wertes erfolgen. Hierbei kann insbesondere Hexahydroxoplatinsäure ausgefällt und auf den Metalloxidpartikeln abgeschieden werden.

Durch Kalzinieren der so erhaltenen Metalloxidpartikel, auf denen die Platinverbindung abgeschieden wurde, kann in einem weiteren Verfahrensschritt elementares Platin auf den Partikeln des Trägermaterials erzeugt werden.

Sowohl Trägermaterial, auf dem die Platinverbindung abgeschieden wurde als auch Trägermaterial, auf dem die Platinverbindung durch Kalzinierung zu metallischem Platin umgesetzt wurde, können zur Herstellung von Autoabgaskatalysatoren verwendet werden.

Hierzu wird eine Beschichtungssuspension (auch als Washcoat bezeichnet) hergestellt, welche das Trägermaterial enthält. Hierbei handelt es sich wie oben beschrieben meist um Metalloxide, insbesondere also Aluminiumoxid oder Siliziumdioxid.

Mit dieser Beschichtungssuspension werden die Innenwände der Strömungskanäle der Tragkörper, welche zur Herstellung der Autoabgaskatalysatoren erforderlich sind, auf an sich bekannte Weise beschichtet, wie z.B. in EP-A1-1273344 beschrieben.

Die Tragkörper werden meist aus Metall oder Keramik hergestellt und können als Durchflußwabenkörper oder Wandflußfilter ausgestaltet sein. Hierbei handelt es sich im Allgemeinen um zylindrische Körper, die eine umlaufende Mantelfläche und zwei gegenüberliegende Stirnflächen aufweisen, wobei die Tragkörper von Strömungskanälen durchzogen sind, die zwischen beiden Stirnflächen verlaufen.

Weit verbreitet sind Katalysatorkörper mit rundem Querschnitt, elliptischem oder dreieckförmigem Querschnitt. Die Strömungskanäle weisen meist einen quadratischen Querschnitt auf und sind in einem engen Raster über den gesamten Querschnitt der Katalysatorkörper angeordnet. Je nach Anwendungsfall variiert die Kanal- beziehungsweise Zelldichte der Strömungskanäle meist zwischen 10 und 250 cm⁻². Für die Abgasreinigung von Personenkraftwagen werden heute noch oft Katalysatortragkörper mit Zelldichten von etwa 62 cm⁻² eingesetzt. Bei Wandflussfiltern sind diese alternierend verschlossen, so dass ein durchfließender Abgasstrom durch die Poren der Wandungen gelenkt wird, welche die Strömungskanäle bilden.

Nach dem Aufbringen der Beschichtungssuspension wird der beschichtete Wabenkörper gegebenenfalls weiteren Verfahrensschritten unterworfen, getrocknet und schließlich kalziniert. Wird Trägermaterial, auf dem die Platinverbindung abgeschieden wurde, verwendet, so findet die Umwandlung in elementares Platin dann bei diesem Kalzinierungsschritt nach Beschichtung des Tragkörpers statt.

Wurde bereits ein nach Abscheidung der Platinverbindung kalziniertes Trägermaterial (wie z.B. Platin auf einem Aluminiumoxid-Trägermaterial) zur Herstellung der Beschichtungssuspension verwendet, so liegt bereits vor der Beschichtung des Tragkörpers elementares Platin auf dem Trägermaterial vor und die Kalzinierung des beschichteten Tragkörpers ist hinsichtlich der Umwandlung der abgeschiedenen Platinverbindung ohne Effekt.

### Beispiele

### Beispiel 1: Herstellung von Pt-TEAH

In einem Reaktor mit einem Füllvolumen von fünf Litern wurden 758 g demineralisiertes Wasser vorgelegt und 1,47 kg wässrige Tetraethylammoniumhydroxidlösung mit einer Konzentration von 35 Gew.-% zugegeben, entsprechend etwas 2 mol pro mol Platin.

Unter Rühren wurden dann 894,74g Hexahydroxoplatinsäure mit einem Gehalt von 38 g Platin zugegeben. Es wurde anschließend bei einer Temperatur von ca. 20°C bis 30°C eine Stunde nachgerührt, wobei eine gelborange Lösung entstand, die über eine G4-Glasfritte filtriert und analysiert wurde.

Die Lösung enthielt einen Edelmetallgehalt von 10,9 Gew.-% Platin und einen pH-Wert von 13,9.

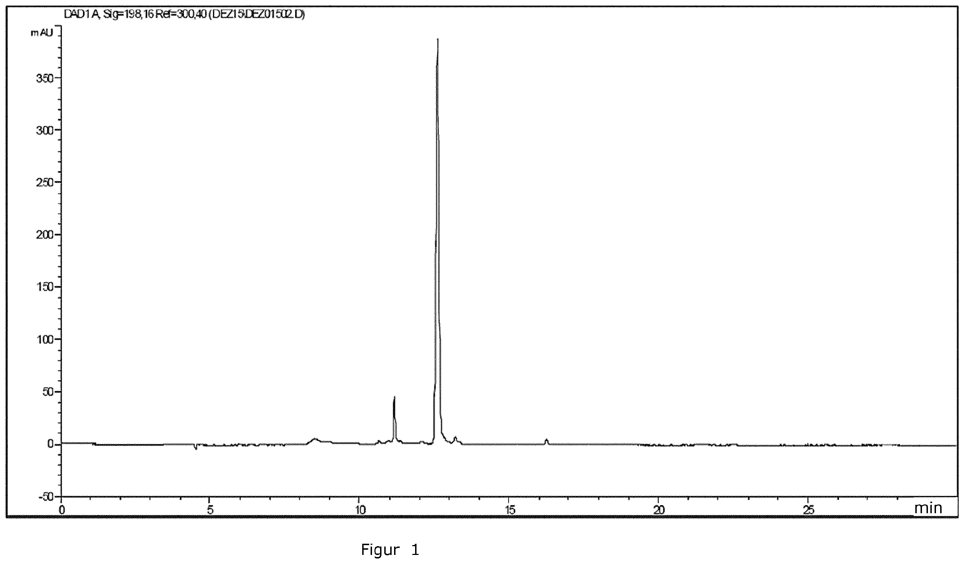

Das Produkt wurde auch durch Kapillarelektrophoreses untersucht, das Ergebnis ist in Figur 1 gezeigt. Die Zusammensetzung ist charakteristisch für die Verbindung Pt-TEAH. Der Hauptpeak entspricht dem [Pt(OH)₆]²⁻ Komplex. Der kleinere, zweite Peak entspricht einem weiteren anionischen Pt-Komplex der unbekannten Zusammensetzung [Pt(OH)₆₋ₓ(NEt)ₓ]^{2-x} (mit x=1-2). Die prozentuale Verteilung beider Hauptkomponenten kann je nach Herstellmethode und Eduktqualität schwanken.

## Patentansprüche

1. Die Verbindung Tetraethylammonium-hexahydroxoplatinat (Pt-TEAH), (N(CH₂CH₃)₄)2[Pt(OH)₆].

2. Verfahren zur Herstellung von Tetraethylammonium-hexahydroxoplatinat (Pt-TEAH) durch Umsetzung von Hexahydroxoplatinsäure mit Tetraethylammoniumhydroxid, optional in Gegenwart eines Reaktionsmediums.

3. Verfahren nach Anspruch 2, wobei Tetraethylammoniumhydroxid oder deren Lösung vorgelegt, Hexahydroxoplatinsäure zugegeben und für eine vorbestimmte Reaktionszeit und bei einer in einem vorbestimmten Bereich liegenden Reaktionstemperatur umgesetzt wird.

4. Verfahren nach Anspruch 2, wobei Hexahydroxoplatinsäure vorgelegt, Tetraethylammoniumhydroxid oder deren Lösung zugegeben und für eine vorbestimmte Reaktionszeit und bei einer in einem vorbestimmten Bereich liegenden Reaktionstemperatur umgesetzt wird.

5. Verfahren nach einem oder mehreren der Ansprüche 2 bis 4, wobei das Reaktionsmedium vor Zugabe der Reaktanden vorgelegt wird.

6. Verfahren nach einem oder mehreren der Ansprüche 2 bis 5, wobei das Reaktionsmedium ein polares, protisches Lösemittel ist.

7. Verfahren nach einem oder mehreren der Ansprüche 2 bis 6, wobei das Reaktionsmedium ausgewählt ist aus der Gruppe bestehend aus Wasser, ein oder mehrere Alkohole, eine oder mehrere Säuren und deren Mischungen.

8. Verfahren nach einem oder mehreren der Ansprüche 2 bis 7, wobei das Reaktionsmedium ein Lösemittel ausgewählt aus der Gruppe bestehend aus Wasser, Methanol, Ethanol, Propanol, Isopropanol, Butanol, 1-Methylbutanol, 2-Methylbutanol, tert.- Butanol, Ameisensäure, Essigsäure, Propionsäure und deren Kombinationen ist.

9. Verfahren nach einem oder mehreren der Ansprüche 2 bis 8, wobei das Verfahren bei einer Temperatur von 10°C bis 100°C, insbesondere 15°C bis 80°C oder 20°C bis 50°C oder 20°C bis 40°C durchgeführt wird.

10. Verfahren nach einem oder mehreren der Ansprüche 2 bis 9, wobei 1,7 bis 17, insbesondere 2 bis 6 Moläquivalente Tetraethylammoniumhydroxid pro Mol Hexahydroxoplatinsäure eingesetzt werden.

11. Wäßrige Lösung von Tetraethylammonium-hexahydroxoplatinat (Pt-TEAH), (N(CH2CH3)4)2[Pt(OH)6] mit einer Platinkonzentration von 1 Gew.-% bis 15 Gew.-%.

12. Verwendung von Tetraethylammonium-hexahydroxoplatinat (Pt-TEAH), (N(CH₂CH₃)₄)₂[Pt(OH)₆], nach Anspruch 1 oder deren wäßrige Zubereitung als Precursor zur Herstellung heterogener Katalysatoren, Autoabgaskatalysatoren, für den Einsatz in galvanischen Bädern, als Vorprodukt für die Herstellung homogener Katalysatoren oder als homogener Katalysator.

13. Verfahren zur Herstellung eines Autoabgaskatalysators, wobei eine Suspension enthaltend Tetraethylammonium-hexahydroxoplatinat, (N(CH₂CH₃)₄)₂[Pt(OH)₆] (Pt-TEAH), und mindestens einem Trägermaterial erzeugt wird, Platin als Hexahydroxoplatinsäure durch Umsetzen von Pt-TEAH auf das Trägermaterial gefällt und ein Katalysator-Tragkörper ganz oder teilweise mit dem Trägermaterial beschichtet wird und die auf das Trägermaterial aufgebrachte Hexahydroxoplatinsäure vor oder nach der Beschichtung des Katalysator-Tragkörpers kalziniert wird.

14. Verfahren nach Anspruch 13, wobei das Trägermaterial ein Metalloxid, insbesondere Aluminiumoxid oder Siliziumdioxid ist.

15. Verfahren nach Anspruch 13 oder 14, wobei der Katalysator-Tragkörper ein Wandflußfilter oder ein Durchflußwabenkörper ist.

16. Verfahren nach einem oder mehreren der Ansprüche 13 bis 15, wobei das Umsetzen von Pt-TEAH zum Fällen von Hexahydroxoplatinsäure auf das Trägermaterial durch Änderung des pH-Wertes erfolgt.

## Claims

1. The compound, tetraethylammonium hexahydroxoplatinate (Pt-TEAH), (N(CH₂CH₃)₄)₂[Pt(OH)₆].

2. Method for producing tetraethylammonium hexahydroxoplatinate (Pt-TEAH) by reacting hexahydroxoplatinic acid with tetraethylammonium hydroxide - optionally, in the presence of a reaction medium.

3. Method according to claim 2, wherein tetraethylammonium hydroxide or its solution is provided, hexahydroxoplatinic acid is added, and a reaction takes place for a predetermined reaction time and at a reaction temperature within a predetermined range.

4. Method according to claim 2, wherein hexahydroxoplatinic acid is provided, tetraethylammonium hydroxide or its solution is added, and a reaction takes place for a predetermined reaction time and at a reaction temperature within a predetermined range.

5. Method according to one or more of claims 2 through 4, wherein the reaction medium is provided before the reactants are added.

6. Method according to one or more of claims 2 through 5, wherein the reaction medium is a polar, protic solvent.

7. Method according to one or more of claims 2 through 6, wherein the reaction medium is selected from the group consisting of water, one or more alcohols, one or more acids, and mixtures thereof.

8. Method according to one or more of claims 2 through 7, wherein the reaction medium is a solvent selected from the group consisting of water, methanol, ethanol, propanol, isopropanol, butanol, 1-methylbutanol, 2-methylbutanol, tert.-butanol, formic acid, acetic acid, propionic acid, and combinations thereof.

9. Method according to one or more of claims 2 through 8, wherein the method is implemented at a temperature of 10 °C to 100 °C - particularly, 15 °C to 80 °C or 20 °C to 50 °C or 20 °C to 40 °C.

10. Method according to one or more of claims 2 through 9, wherein 1.7 to 17 - particularly, 2 to 6 - molar equivalents of tetraethylammonium hydroxide are used per mole of hexahydroxoplatinic acid.

11. Aqueous solution of tetraethylammonium hexahydroxoplatinate (Pt-TEAH), (N(CH₂CH₃)₄)2[Pt(OH)₆] with a platinum concentration of 1 wt% to 15 wt%.

12. Use of tetraethylammonium hexahydroxoplatinate (Pt-TEAH), (N(CH₂CH₃)₄)₂[Pt(OH)₆], according to claim 1 or the aqueous preparation thereof as a precursor for producing heterogeneous catalysts, automotive exhaust gas catalysts, for use in galvanic baths, as a precursor for the production of homogeneous catalysts, or as a homogeneous catalyst.

13. Method for producing an automotive exhaust gas catalyst, wherein a suspension containing tetraethylammonium hexahydroxoplatinate, (N(CH₂CH₃)₄)2[Pt(OH)₆] (Pt-TEAH), and at least one support material is created, platinum precipitates onto the support material as hexahydroxoplatinic acid by reacting Pt-TEAH, and a catalyst support body is completely or partially coated with the support material, and the hexahydroxoplatinic acid applied to the support material is calcined before or after the coating of the catalyst support body.

14. Method according to claim 13, wherein the support material is a metal oxide - in particular, aluminum oxide or silicon dioxide.

15. Method according to claim 13 or 14, wherein the catalyst support body is a wall-flow filter or a through-flow honeycomb body.

16. Method according to one or more of claims 13 through 15, wherein the reaction of Pt-TEAH for the precipitation of hexahydroxoplatinic acid onto the support material takes place by changing the pH value.

## Revendications

1. Composé hexahydroxoplatinate de tétraéthylammonium (Pt-TEAH), (N(CH₂CH₃)₄)₂[Pt(OH)₆].

2. Procédé de préparation d'hexahydroxoplatinate de tétraéthylammonium (Pt-TEAH) par réaction d'acide hexahydroxoplatinique avec de l'hydroxyde de tétraéthylammonium, facultativement en présence d'un milieu de réaction.

3. Procédé selon la revendication 2, dans lequel on dispose au préalable l'hydroxyde de tétraéthylammonium ou sa solution, on ajoute l'acide hexahydroxoplatinique et on fait réagir pendant un temps de réaction prédéfini et à une température de réaction se trouvant dans une plage prédéfinie.

4. Procédé selon la revendication 2, dans lequel on dispose au préalable l'acide hexahydroxoplatinique, on ajoute l'hydroxyde de tétraéthylammonium ou sa solution et on fait réagir pendant un temps de réaction prédéfini et à une température de réaction se trouvant dans une plage prédéfinie.

5. Procédé selon l'une ou plusieurs des revendications 2 à 4, dans lequel le milieu de réaction est disposé au préalable, avant l'ajout des réactifs.

6. Procédé selon l'une ou plusieurs des revendications 2 à 5, dans lequel le milieu de réaction est un solvant polaire protique.

7. Procédé selon l'une ou plusieurs des revendications 2 à 6, dans lequel le milieu de réaction est choisi dans le groupe constitué de l'eau, d'un ou de plusieurs alcools, d'un ou de plusieurs acides et de leurs mélanges.

8. Procédé selon l'une ou plusieurs des revendications 2 à 7, dans lequel le milieu de réaction est un solvant choisi dans le groupe constitué de l'eau, du méthanol, de l'éthanol, du propanol, de l'isopropanol, du butanol, du 1-méthylbutanol, du 2-méthylbutanol, du tert-butanol, de l'acide formique, de l'acide acétique, de l'acide propionique ou de leurs combinaisons.

9. Procédé selon l'une ou plusieurs des revendications 2 à 8, dans lequel le procédé est réalisé à une température de 10 °C à 100 °C, en particulier de 15 °C à 80 °C ou de 20 °C à 50 °C ou de 20 °C à 40 °C.

10. Procédé selon l'une ou plusieurs des revendications 2 à 9, dans lequel 1,7 à 17, en particulier 2 à 6, équivalents molaires d'hydroxyde de tétraéthylammonium par mole d'acide hexahydroxoplatinique sont utilisés.

11. Solution aqueuse d'hexahydroxoplatinate de tétraéthylammonium (Pt-TEAH), (N(CH₂CH₃)₄)₂[Pt(OH)₆], présentant une concentration en platine de 1 % en poids à 15 % en poids.

12. Utilisation d'hexahydroxoplatinate de tétraéthylammonium (Pt-TEAH), (N(CH₂CH₃)₄)2[Pt(OH)₆] selon la revendication 1 ou de sa préparation aqueuse en tant que précurseur pour la préparation de catalyseurs hétérogènes, de catalyseurs des gaz d'échappement automobiles, pour l'utilisation dans des bains galvaniques, en tant que précurseur pour la préparation de catalyseurs homogènes ou en tant que catalyseur homogène.

13. Procédé de préparation d'un catalyseur de gaz d'échappement automobiles, dans lequel une suspension contenant de l'hexahydroxoplatinate de tétraéthylammonium, (N(CH₂CH₃)₄)₂[Pt(OH)₆], (Pt-TEAH), et au moins un matériau support est produite, du platine est précipité sur le matériau support en tant qu'acide hexahydroxoplatinique par transformation de Pt-TEAH et un corps support de catalyseur est totalement ou partiellement revêtu par le matériau support et l'acide hexahydroxoplatinique appliqué sur le matériau support est calciné avant ou après le revêtement du corps support de catalyseur.

14. Procédé selon la revendication 13, dans lequel le matériau support est un oxyde métallique, en particulier l'oxyde d'aluminium ou l'oxyde de silicium.

15. Procédé selon la revendication 13 ou 14, dans lequel le corps support de catalyseur est un filtre à parois filtrantes ou un corps en nid d'abeille à écoulement.

16. Procédé selon l'une ou plusieurs des revendications 13 à 15, dans lequel la transformation du Pt-TEAH pour la précipitation de l'acide hexahydroxoplatinique sur le matériau support est effectuée par modification de la valeur de pH.
